# EUROPEAN PATENT APPLICATION

(11) **EP 1 656 950 A1**
(43) Date of publication of application: **17.05.2006**
(21) Application number: 04017939.2
(22) Date of filing: 08.04.1993
(51) Int. Cl.: A61K 39/395

(54) **Immunotoxins directed against CD33 related surface antigens**

(30) Priority: 10.04.1992 US 866693
(62) Divisional of application: 93912143.0
(71) Applicant: RESEARCH DEVELOPMENT FOUNDATION, Carson City, Nevada 89703 (US)
(72) Inventor: Rosenblum, Michael G., Houston, Texas 77071 (US)
(74) Representative: McQueen, Andrew Peter

(57) **Abstract**

The present invention provides novel immunotoxins and methods of treating neoplastic diseases. These immunotoxins are comprised of a conjugation of an antigen binding region exhibiting binding specificity for the CD33 protein and a cell growth modulator. The immunotoxins of the present invention specifically and selectively kill tumor cells that are characterized by the expression of CD33 antigen. Thus, the novel immunotoxins would be useful in treating human leukemias, both acute and chronic, and other myelodysplastic syndromes.

## Description

### BACKGROUND OF THE INVENTION

### Field of the Invention

The present invention relates generally to the field of treatment of neoplastic disease. More specifically, the present invention relates to novel immunoconjugates and their use in the treatment of neoplastic disease. Even more particularly, the present invention relates to novel immunoconjugates cytotoxic to leukemia cells characterized by expression of the CD33 antigen.

### Description of the Related Art

Neoplastic disease is one of the leading causes of mortality and morbidity in the Western World. All neoplastic diseases or "cancers" share at least one characteristic, i.e., the involvement of defects in the cellular growth regulatory process.

Antigens located on the surface of cancer cells have been useful in distinguishing lymphoid from non-lymphoid leukemias, subtyping of acute myelogenous leukemia, predicting therapeutic outcome and in therapy *in vivo* or via a bone marrow purging *ex vivo.* Antigens defining acute non-lymphocytic cells also identify normal hematopoietic cells during early stages of their development.

CD33 antigen is a 67 kilodalton glycoprotein found on normal colony forming unit granulocyte-monocyte (CFU-GM), on a fraction of burst-forming unit-erythroid (BFU-E and CFU-granulocyte, erythroid, monocyte, megakaryocyte) CFU-GEMM, and absent from normal pluripotent stem cells.

Antibodies are proteins normally produced by the immune system of an animal in response to antigenic determinants. Antibodies bind to the specific antigen to which they are directed. The development of specific monoclonal antibodies has provided investigators with a possible means of selectively targeting chemotherapeutic agents to cells which overexpress tumor associated antigens.

Immunotoxins are hybrid molecules consisting of a monoclonal antibody covalently linked to a toxin. Immunotoxins have several possible advantages over conventional anti-neoplastic agents including selectivity for tumor cells and potential delivery of extremely potent toxins.

The presence of the CD33 antigen on acute non-lymphoid leukemic cells and acute myelogenous leukemic cells presents an opportunity for development of selective immunotoxins. Currently, no such effective immunotoxins exist. Thus, there continues to exist a great need and desire in this art for compounds and methods of selectively killing leukemia cells.

### SUMMARY OF THE INVENTION

The present invention provides a novel composition comprising a conjugate of an antigen binding region exhibiting binding specificity for the CD33 protein and a cell growth modulator. Such a composition acts as an immunotoxin to specifically kill tumor cells characterized by the expression of the CD33 protein.

Thus, in one embodiment of the present invention, there is provided a new composition of matter comprising a conjugate of an antigen binding region exhibiting binding specificity for the CD33 protein and a cell growth modulator. The cell growth modulator may be a toxin, a cytocidal drug, a cytostatic drug, or a biological response modifier. Preferably, the cell growth modulator is gelonin.

In another embodiment of the present invention, there is provided a method of treating neoplastic disease comprising the administration of a cytocidally effective dose of an immunotoxin of the present invention to an individual in need of such treatment.

And yet another embodiment of the present invention, there is provided a method of killing tumor cells in bone marrow comprising removing bone marrow from an individual having a neoplastic disease, treating the bone marrow with a composition of the present invention and infusing the treated bone marrow back into the individual.

In another embodiment of the present invention there is provided a method of preventing recurrence of neoplastic disease where the disease is characterized by an expression of CD33 protein. The recurrence is prevented by administration of a cytocidally effective treatment of immunotoxins of the present invention.

In still another embodiment of the present invention, there is provided a new composition of matter comprising a fusion protein formed by the fusion of the CD33 antigen binding region and a cell growth modulator. Preferably, the cell growth modulator is gelonin.

In further embodiments of the present invention there are provided methods of extending the survival time of a mammal bearing tumor by administration of the immunotoxin of the present invention to this mammal. In yet further embodiments, there are provided a method of retarding the rate of growth of tumors by administering the immunotoxin of the present invention. Still further, there is provided a pharmaceutical composition comprising an immunotoxin of the present invention.

### BRIEF DESCRIPTION OF THE DRAWINGS

Figure 1 depicts the inhibition of protein synthesis by gelonin and the M195 immunotoxin on HL60 cells.
Figure 2 demonstrates the inhibition of protein synthesis by gelonin and M195 gelonin on SKLY16 and HL60 cell lines.
Figure 3 illustrates the inhibition of protein synthesis by a three day incubation of M195 immunotoxin with HL60.
Figure 4 depicts the inhibition of protein synthesis by a five day incubation of M195-IT on HL60 cells.
Figure 5 demonstrates the binding of HuG1 and HuG1-IT to HL60, U937, MOLT4 cells.
Figure 6 illustrates the inhibition of DNA synthesis resulting from HuG1-IT on HL60 cells.
Figure 7 depicts the competition between HuG1-IT and HuG1 or FD79.
Figure 8 illustrates the electrophoretic pattern of M195 alone, a reaction mixture, gelonin alone, or purified M195 gelonin.

### DETAILED DESCRIPTION OF THE INVENTION

As used herein, the term "chimeric antibodies" or "chimeric peptides" refer to those antibodies or antibody peptides wherein one portion of the peptide has an amino acid sequence that is derived from, or is homologous to, a corresponding sequence in an antibody or peptide derived from a first gene source, while the remaining segment of the chain(s) is homologous to corresponding sequences of another gene source. For example, a chimeric heavy chain antibody peptide may comprise a murine variable region and a human constant region. The two gene sources will typically be two separate species, but will occasionally involve one species.

Chimeric antibodies or peptides are typically produced using recombinant molecular and/or cellular techniques. In many cases, chimeric antibodies have variable regions of both light and heavy chains that mimic the variable regions of antibodies derived from one mammalian species, while the constant and/or framework portions are homologous to the sequences in antibodies derived from a second, different mammalian species.

As used herein, the definition of chimeric antibody, however, is not limited to this example. A chimeric antibody is any antibody in which either or both of the heavy or light chains are composed of combinations of sequences mimicking the sequences in antibodies of different sources, whether these sources be from differing classes, differing antigen responses, or differing species of origin, and whether or not the fusion point is at the variable/constant boundary. For example, chimeric antibodies can include antibodies where the framework and complementarity-determining regions (CDRs) are from different sources. For example, non-human CDRs are integrated into human framework regions linked to a human constant region to make "humanized antibodies." *See, e.g.*, PCT Application Publication No. WO 87/02671; U.S. Patent No. 4,816,567; EP Patent Application 0173494; Jones, *et al.,* Nature, 321:522-525 (1986); and Verhoeyen, *et al.*, *Science,* 239:1534-1536 (1988), all of which are incorporated by reference herein.

As used herein, the term "human-like framework region" is a framework region for each antibody chain, and it usually comprises at least about 70 or more amino acid residues, typically 75 to 85 or more residues. The amino acid residues of the human-like framework region are at least about 80%, preferably about 80-85%, and most preferably more than 85% homologous with those in a human immunoglobulin. This shared feature with other endergenous antibodies is useful in generating a targeting moiety which introduces only a minor immune reaction, *e.g.*, a mechanism which minimizes response to "self" markers.

As used herein, the term "humanized" or "human-like immunoglobulin" refers to an immunoglobulin comprising a human-like framework region and a constant region that is substantially homologous to a human immunoglobulin constant region, *e.g.*, having at least about 80% or more, preferably about 85-90% or more and most preferably about 95% or more homology. Hence, most parts of a human-like immunoglobulin, except possibly the CDRs, are substantially homologous to corresponding parts of one or more native human immunoglobulin sequences.

As used herein, the term "hybrid antibody" refers to an antibody wherein each chain is separately homologous with reference to a mammalian antibody chain, but the combination represents a novel assembly so that two different antigens are recognized by the antibody. In hybrid antibodies, one heavy and light chain pair is homologous to that found in an antibody raised against one antigen recognition feature, *e.g.,* epitope, while the other heavy and light chain pair is homologous to a pair found in an antibody raised against another epitope. This results in the property of multi-functional valency, i.e., ability to bind at least two different epitopes simultaneously. Such hybrids may, of course, also be formed using chimeric chains.

As used herein, the terms "monoclonal antibody" means an antibody composition recognizing a discrete antigen determinant. It is not intended to be limited as regards the source of the antibody or the manner in which it is made.

For this invention, an antibody or other peptide is specific for a CD33 if the antibody or peptide binds or is capable of binding CD33, *e.g.,* protein as measured or determined by standard antibody-antigen or ligand-receptor assays, *e.g.*, competitive assays, saturation assays, or standard immunoassays such as ELISA or RIA. This definition of specificity applies to single heavy and/or light chains, CDRs, fusion proteins or fragments of heavy and/or light chains, that are also specific for CD33 if they bind CD33 alone or if, when properly incorporated into immunoglobulin conformation with complementary variable regions and constant regions as appropriate, are then capable of binding CD33 with specificity.

In competition assays the ability of an antibody or peptide fragment to bind an antigen can be determined by detecting the ability of the peptide to compete with the binding of a compound known to bind the antigen. Numerous types of competitive assays are known and are discussed herein. Alternatively, assays that measure binding of a test compound in the absence of an inhibitor may also be used. For instance, the ability of a molecule or other compound to bind the c-erbB-2 protein can be detected by labelling the molecule of interest directly or it may be unlabelled and detected indirectly using various sandwich assay formats. Numerous types of binding assays such as competitive binding assays are known (*see, e.g.,* U.S. Patent Nos. 3,376,110, 4,016,043, and Harlow and Lane, *Antibodies: A Laboratory Manual,* Cold Spring Harbor Publications, N.Y. (1988)) which are incorporated herein by reference). Assays for measuring binding of a test compound to one component alone rather than using a competition assay are also available. For instance, immunoglobulins can be used to identify the presence of the CD33. Standard procedures for monoclonal antibody assays, such as ELISA, may be used (*see*, Harlow and Lane, *supra*). For a review of various signal producing systems which may be used. *See,* U.S. Patent No. 4,391,904, which is incorporated herein by reference.

Further, the specificity of the binding moieties to CD33 can be determined by their affinity. Such specificity exists if the dissociation constant (K_{D} = 1/K, where K is the affinity constant) of the moiety is < 1µM, preferably < 100 nM, and most preferably < 1 nM. Antibody molecules will typically have a K_{D} in the lower ranges. K_{D} = [R-L]/[R][L] where [R], [L], and [R-L] are the concentrations at equilibrium of the receptor or CD33 (R), ligand, antibody, or peptide (L) and receptor-ligand complex (R-L), respectively. Typically, the binding interactions between ligand or peptide and receptor or antigen include reversible noncovalent associations such as electrostatic attraction, Van der Waals forces, and hydrogen bonds.

Other assay formats may involve the detection of the presence or absence of various physiological or chemical changes that result from the interaction, such as down modulation, internalization, or an increase in phosphorylation, as described in United States Patent Application No. 07/644,361 filed 1/18/91, incorporated by reference herein. *See also, Receptor-Effector Coupling - A Practical Approach,* ed. Hulme, IRL Press, Oxford (1990).

Gelonin is a glycoprotein (M.W. approximately 29-30,000 Kd) purified from the seeds of *Gelonium multiforum.* Gelonin belongs to a class of potent ribosomal-inactivating plant toxins. Other members of this class of ribosomal-inactivating plant toxins are the chains of abrin, ricin and modeccin. Gelonin, like abrin and ricin, inhibits protein synthesis by damaging the 60S sub-unit of mammalian ribosomes. Gelonin appears to be stable to chemical and physical treatment. Furthermore, gelonin itself does not bind to cells and, therefore, is non-toxic (except in high concentrations) and is safe to manipulate in the laboratory. The inactivation of ribosomes is irreversible, does not appear to involve co-factors and occurs with an efficiency which suggests that gelonin acts enzymatically.

Gelonin and ricin are among the most active toxins which inhibit protein synthesis on a protein weight basis. Gelonin is 10 to 1000 times more active in inhibiting protein synthesis than ricin A chain. Peptides like ricin and abrin are composed of two chains, an A chain which is the toxic unit and a B chain which acts by binding to cells. Unlike ricin and abrin, gelonin is composed of a single chain, and, because it lacks a B chain for binding to cells, it is itself relatively non-toxic to intact cells.

Mammalian cells apparently lack the ability to bind and/or to internalize the native gelonin molecule. Conjugates of gelonin with monoclonal antibody, such as M195 directed to a tumor associated antigen present on certain tumor cells, provide both a specific method for binding the gelonin to the cell and a route for internalization of the gelonin-antibody complex.

The cytotoxic moiety of the immunotoxin may be a cytotoxic drug or an enzymatically active toxin of bacterial or plant origin, or an enzymatically active fragment ("A chain") of such a toxin. Enzymatically active toxins and fragments thereof are preferred and are exemplified by gelonin, diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytoiacca americana* proteins (PAPI, PAPII, and PAP-S), momordica charantia inhibitor, curcin, crotin, saponaria officinalis inhibitor, mitogellin, restrictocin, phenomycin, and enomycin. Most preferred is the conjugation with gelonin.

Active fragments and derivatives include any compounds which have the same core structure as the full length structure of gelonin but lack the entire primary sequence. These fragments or derivatives will have the same or improved biological or cytotoxic activity as gelonin. The cytotoxicity of the gelonin fragments or derivatives may be routinely determined by those with skill in the art using the rabbit reticulocyte lysate assay.

Biological response modifiers which may be coupled to the M195 antibody and used in the present invention include, but are not limited to, lymphokines and cytokines such as IL-1, IL-2, interferons (α, β, or T) TNF, LT, TGF-β, and IL-6. These biological response modifiers have a variety of effects on tumor cells. Among these effects are increased tumor cell killing by direct action as well as increased tumor cell killing by increased host defense mediated processes. Conjugation of antibody M195 to these biological response modifiers will allow selective localization within tumors and, hence, improved anti-proliferative effects while suppressing non-specific effects leading to toxicity of non-target cells.

Cytotoxic drugs (and derivatives thereof) which are useful in the present invention include, but are not limited to, adriamycin, cis-platinum complex, bleomycin and methotrexate. These cytotoxic drugs are useful for clinical management of recurrent tumors, but their use is complicated by severe side effects and damage caused to non-target cells. The M195 antibody may serve as a useful carrier of such drugs providing an efficient means of both delivery to the tumor and enhanced entry into the tumor cells themselves. In addition, specific antibody delivery of cytotoxic drugs to tumors will provide protection of sensitive sites such as the liver that do not express CD33 and bone marrow stem cells from the deleterious action of the chemotherapeutic agents. Use of drugs conjugated to the M195 antibody as a delivery system allows lower dosage of the drug itself, since all drug moieties are conjugated to antibodies which concentrate within the tumor or leukemia.

Conjugates of the monoclonal antibody may be made using a variety of bifunctional protein coupling agents. Examples of such reagents are SPDP, iminothiolane (IT), bifunctional derivatives of imidoesters such as dimethyl adipimidate, HCl, active esters such as disuccinimidyl suberate, aldehydes such as glutaraldehyde, bis-azido compounds such as bis(p-azidobenzoyl) hexanediamine, bis-diazonium derivatives such as bis-(p-diazoniumbenzoyl)-ethylenediamine, diisocyanates such as tolylene 2,6-diisocyanate, and bis-active fluorine compounds such as a 1,5-difluoro-2,4-dinitrobenzene.

Administration of the immunotoxins of the present invention to an individual who has been diagnosed as having a leukemia that is characterized by the expression of CD33 protein will allow targeting and concentration of the cytotoxic agent at the site where it is needed to kill the tumor cells. By so targeting the cytotoxic agents, non-specific toxicity to other organs, tissues and cells will be eliminated or decreased.

When used *in vivo* for therapy, the immunotoxins are administered to the human or animal patient in therapeutically effective amounts, i.e., amounts that eliminate or reduce the tumor burden or in amounts to eliminate residual disease after an earlier treatment with chemotherapy or radiation therapy. They will normally be administered parenterally, preferably intravenously. The dose and dosage regimen will depend upon the nature of the leukemia and its population, the characteristics of the particular immunotoxin, *e.g.,* its therapeutic index, the patient, and the patient's history. The amount of immunotoxin administered will typically be in the range of about 0.01 to about 1.0 mg/kg of patient weight.

For parenteral administration the immunotoxins will be formulated in a unit dosage injectable form (solution, suspension, emulsion) in association with a pharmaceutically acceptable parenteral vehicle. Such vehicles are inherently non-toxic and non-therapeutic. Examples of such vehicles are water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances that enhance isotonicity and chemical stability, *e.g.,* buffers and preservatives. The immunotoxin will typically be formulated in such vehicles at concentrations of about 0.1 mg/ml to 10 mg/ml.

The immunotoxins of the present invention may also be used in a method of killing tumor cells in bone marrow. In this method, the bone marrow is first removed from an individual having a neoplastic disease such as leukemia. Subsequently, the bone marrow is treated with a cytocidally effective dose of an immunotoxin of the present invention.

The following examples provide a detailed description of the preparation, characterization, and use of the immunotoxins of this invention. These examples are not intended to limit the invention in any manner.

### Example 1

### Purification of Gelonin

Seeds of *Gelonium multiflorum* were shelled and the nuts ground in a homogenizer with eight volumes of 0.14 M NaCl containing a 5 mM sodium phosphate (pH 7.4). The homogenate was left overnight at 4°C with continuous stirring, cooled on ice and centrifuged at 35,000 times g for 20 minutes at 0°C. The supernatant was removed, dialyzed against 5 mM sodium phosphate (pH 6.5) and concentrated using a pm10 filter. The sample was layered on a CM-52 ion-exchange column (20 x 1.5 cm) equilibrated with 5 mM sodium phosphate (pH 6.5). Material which bound to the ion exchange resin was eluted with 400 ml of 0 to 0.3 M linear NaCl gradient at a rate of 25 ml per hour at 4°C. Five ml fractions were collected. The fractions were monitored at 280 nm in a spectrophotometer. The gelonin eluted in about fractions 55-70 and was the last major elution peak. Fractions 55-70 were pooled, dialyzed against double distilled water and concentrated by lyophilization. The purity and the molecular weight of each preparation was checked on high pressure liquid chromatography using a TSK 3000 gel permeation column with 50 mM sodium phosphate buffer, pH 7.4 and 15% sodium dodecylsulphate-polyacrylamide gel electrophoresis (SDS-page). Gelonin migrated as a single band with an approximate molecular weight of 29-30,000 daltons.

### Example 2

### Assay of Gelonin Activity

The gelonin activity was monitored in a cell-free protein synthesis inhibition assay. The cell-free protein synthesis inhibition assay was performed by sequentially adding to 50 µl rabbit reticulocyte lysate, mixing after each addition, the following components: 0.5 ml of 0.2 M Tris-HCl (pH 7.8), 8.9 ml of ethylene glycol, and 0.25 ml of 1 M HCl).

Twenty microliters of a salt-amino acid-energy mixture (SAEM) consisting of: 0.375 M KCl, 10 mM Mg(CH₃CO₂)₂, 15 mM glucose, 0.25-10 mM amino acids (excluding leucine), 5 mM ATP, 1 mM GTP, 50 mM Tris-HCl (pH 7.6), 10 µl Creatinine phosphate-creatinine phosphokinase, 8 µl ¹⁴C leucine (Amersham, 348 mCi mmol), and adding 1.5 µl of solutions containing varying concentrations of the gelonin mixture. The mixture was incubated for 60 minutes at 30°C. ¹⁴C-leucine incorporation was monitored in an aliquot of the mixture by precipitating synthesized protein on glass fiber filters, washing in 10% TCA and acetone, and monitoring the radioactivity in a Beta-counter using Aquasol scintillation fluid. Gelonin with a specific activity no lower than 4 x 10⁹ U/mg was used for conjugation with the antibodies. A unit of gelonin activity is the amount of gelonin protein which causes 50% inhibition of incorporation of [¹⁴C] leucine into protein in the cell free assay.

### Example 3

### Modification of Gelonin With Iminothiolane

### Preparation of 2-IT Modified Gelonin

Gelonin in phosphate buffered saline was concentrated to approximately 10 mg/ml in a Centriprep 10 concentrator. Triethanolamine hydrochloride (TEA/HCl), pH 8.0, and EDTA were added to a final concentration of 60 mM TEA/HCl and 1 mM EDTA, pH 8.0. A 2-iminothiolane stock solution (500 mM in 60 mM TEA/HCl buffer containing 1 mM EDTA, pH 8.0) was added to a final concentration of 1 mM and the sample was incubated for 90 min at 4°C under a stream of nitrogen gas with stirring. Excess iminothiolane was removed by gel filtration on a column of Sephadex G-25 (1 x 24 cm) pre-equilibrated with phosphate-EDTA buffer, pH 7.5, containing 0.01 M Na₂HPO₄, 0.0018 M KH₂PO₄, 0.0034 M KCl, 0.001 M EDTA and 0.17 M NaCl. Fractions were analyzed for protein content in microtiter plates using Bio-Rad assay. Gelonin eluted at the void volume (about fractions 21-23). These fractions were pooled and stored at 4°C.

M195 linked with 4-succinimidyloxycarbonyl-α-methyl-α(2-pyridyldithio)toluene (SMPT) is prepared by coupling 2-IT-modified gelonin with SMPT-modified MAB M195. Briefly, to modify M195 with SMPT, 10 mg of antibody in 1.0 ml of PBS is diluted 1:1 with 2X borate buffer (0.05 M sodium borate 1.7% sodium chloride, pH 9.0) and 52 µl of 4 mM SMPT in dry DMF is slowly added to the antibody solution. The reaction is incubated at room temperature for 2 hr with stirring under N₂. Excess SMPT is removed by passing the reactions mixture through a Sephadex G-25 column containing phosphate-EDTA buffer, pH 7.5, and antibody positive fractions are evaluated by Bio-Rad assay. The fractions are pooled and stored at 4°C under N₂. The cross-link with 2-IT is carried out at 27°C under N₂ with stirring for 96 hrs. The final product is purified as previously described for SPDP.

### Example 4

### Preparation of Murine Monoclonal Antibody M195

Murine monoclonal antibody M195 was produced from hybridomas resulting from the fusion of NS-1 mouse myeloma cells and the spleen cells of a five week old BALB/c mouse immunized with leukemia cells from a patient with acute non-lymphocytic leukemia (FAB-M2). Supernatant fluids from cloned hybridoma cultures were screened against the panel of leukemia cell lines and the original ANLL leukemia cells using *Staphylococcus aureus* protein A (PA) erythrocyte resetting. The repeatedly subcloned M195 hybridoma was expanded in the doubly pristane-primed (C57 BL/6 times BALB/c) F1 mice.

M195 was purified on a PA-Sepharose by affinity chromatography using sequential PH step dilutions. Purity was determined on a sodium dodecyl (SDS) -polyacrylamide gels stained with coomassie brilliant blue. Humanized monoclonal antibody M195 was prepared as described by Co *et al.,* "Chimeric and Humanized Antibodies with Specificity for the CD33 Antigen", *J. Immunol.,* 148:1149-1154 (1992) and was produced from hybridomas and purified as above.

Briefly, the hypervariable domain (V region) for the H and L chains for murine M195 were cloned by an anchored PCR method. First, M195 hybridoma cells were lysed and the RNA was extracted using the hot phenol method. cDNA was synthesized from the RNA by incubation with viral reverse transcriptase. PCR primers were then designed and both ECO RI and Hind III sites were included in the upstream and downstream primers for convenience subcloning. The PCR reactions were performed in a programmable heating block using 30 rounds of temperature cycling (92°C for 1 minute, 50°C for 2 minutes and 72°C for 3 minutes). The PCR products were separated by electrophoresis on low melting point agarose gel. The bands were excised, digested with restriction enzymes and cloned into the PUC 18 vector for sequence determination. The murine H and L variable regions were then spliced into plasmids containing complementary human H and L chains using different vectors. The vectors encoding chimeric mouse/human L and H chains were then transfected into SP 2/0 cells by electroporation. Surviving colonies were plated and tested for ability to produce humanized αCD₃₃ antibody.

### Example 5

### Conjugation of SPDP-Modified Monoclonal Antibody M195 With Iminothiolane-modified Gelonin

One milligram of purified gelonin (2 mg/ml in PBS) prepared as described in Example 1 was modified with iminothiolane as described in Example 3. Monoclonal antibody M195 modified as described in Example 4 was mixed with an equal weight of the modified gelonin. This proportion corresponded to a 5-fold molar excess of gelonin as compared to antibody. The pH of the mixture was adjusted to 7.0 by the addition of 0.05 M TEA/HCl buffer pH 8.0 and the mixture was incubated for 20 hours at 4°C under nitrogen. Iodoacetamide (0.1 M) was added to a final concentration of 2 mM to block any remaining free sulfhydryl groups and incubation was continued for an additional hour at about 25°C. The reaction mixture was stored at 4°C until purification by gel filtration.

### Example 6

### Purification of Gelonin-Monoclonal Antibody M195 Complexes

Non-conjugated gelonin and low molecular weight products were removed from the reaction mixtures of Example 6 by gel filtration on a Sephadex S-300 column (1.6 x 31 cm) pre-equilibrated with PBS.

Reaction mixtures from Example 5 were concentrated to approximately 1 ml with a Centricon 30 microconcentrator before loading on the Sephadex column. The column was washed with PBS. One ml fractions were collected and 50 µl aliquots are analyzed for protein by the Bradford assay.

To remove unconjugated M195, the high molecular weight peak (fraction 28-40) from the S-300 column was applied to an affinity chromatography column of Blue Sepharose CL-6B (1 x 24 cm) pre-equilibrated with 10 mM phosphate buffer (pH 7.2) containing 0.1 M NaCl. After sample loading the column was washed with 30 ml of buffer to completely elute non-conjugated antibody. The column was eluted with a linear salt gradient of 0.1 to 2 M Nacl in 10 mM phosphate buffer pH 7.2. Protein content of the eluted fractions was determined by the Bradford assay.

Non-conjugated antibody was removed from the gelonin conjugated antibody by affinity chromatography on a column (1 x 24 cm) of Blue Sepharose CL-6B pre-equilibrated with 10 mM phosphate buffer, pH 7.2 containing 0.1 M NaCl. After loading the S-300 eluate sample, the column was washed with 30 ml of the same buffer to completely elute non-conjugated antibody.

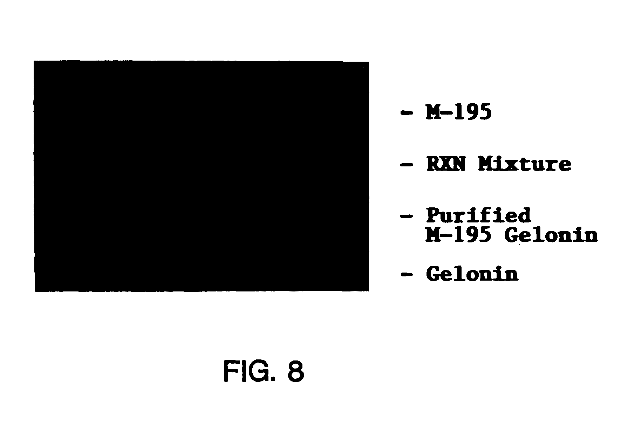

Gelonin-conjugated antibody bound to the column and was eluted with a linear salt gradient of 0.2 to 2 M NaCl in 10 mM phosphate buffer, pH 7.2. The antibody-gelonin complex eluted at approximately 0.7 M NaCl. Protein content of the eluted fractions was determined by the Bradford assay. The protein-containing fractions were pooled and the elution pattern confirmed by electrophoresis on a 5 to 20% gradient non-reducing polyacrylamide gel. Figure 8 illustrates the electrophoretic pattern of M195 alone, an unpurified reaction mixture of M195, gelonin and M195-gelonin, gelonin alone, or purified M195-gelonin. The flow-through peak (fractions 14-20) contains only free antibody while fractions 50-80, eluted with high salt, contain M195-gelonin conjugate free of unconjugated gelonin or antibody. The final product contained M195 antibody coupled to 1, 2 or 3 gelonin molecules. Average gelonin content was 1.5 molecules per antibody molecule. The rabbit reticulocyte *in vitro* translation system was utilized to estimate the gelonin activity of the essentially pure gelonin M195 antibody complex. One unit of activity in this assay was defined as the amount of protein required to provide 50% inhibition of protein synthesis as compared to untreated controls. Utilizing this assay, the specific activity of both the native gelonin and the M195-gelonin conjugate were determined to be 2 x 10⁸ U/mg and 8.2 x 10⁵ U/mg, respectively. The essentially pure gelonin M195 antibody is active in the reticulocyte lysate assay. A 1:1000 dilution of the original sample caused approximately a 50% inhibition of protein synthesis, i.e., a 50% reduction of the incorporation of ¹⁴C-leucine into protein. Thus, the activity of the original preparation was 1000 U/ml.

### Example 7

The compositions of the present invention may include fusion constructs of the M195 monoclonal antibody and a cytotoxic moiety. Such fusion constructs of the immunotoxin of the present invention may be prepared by the method of Co, *et al.,* incorporated herein by reference.

Prior to use in these studies, the Sp2/0-Ag14 cells will be grown initially in the presence of 0.1 µg/ml of native gelonin. Over several months, the concentration of gelonin will be gradually increased until the cells can be maintained in up to 10 mg/ml. Cells will then be cloned by limiting dilution in the presence of 10 mg/ml gelonin and the resulting colonies resistant to gelonin will be expanded. Gelonin will then be removed from the culture media for two passages and the cells challenged again with gelonin exposure to confirm development of stably-resistant clones. After tests to confirm the production and activity of humanized M195, gelonin-resistant SP2/0 cell producing antibody will be grown and the cDNA for the M195 antibody removed from the total DNA by incubation with restriction endonuclease. In parallel, the cDNA from JM105 *E. coli* expressing optimized gelonin will be removed, purified and the DNA encoding gelonin released after digestion with HindIII and Eco RI. The gelonin gene will be ligated into the heavy-chain fragment and the insert replaced into gelonin resistant SP2/0 cells. Cells will then be sub-cloned by limiting dilution and the clones screened for both humanized antibody production and gelonin content. Finally, positive clones will be expanded and the recombinant fusion protein will be purified and tested in both *in vitro* cytotoxicity assays and *in vivo* tissue distribution, pharmacokinetics, therapeutics and toxicity trials. A comparison of M195 gelonin fusion protein properties to the characteristics of the previously described M195-gelonin constructs will be performed to determine the advantages and drawbacks of each. Based upon these studies a Phase I clinical study of chimeric M195-gelonin fusion protein may be performed in patients with advanced breast cancer.

### Example 8

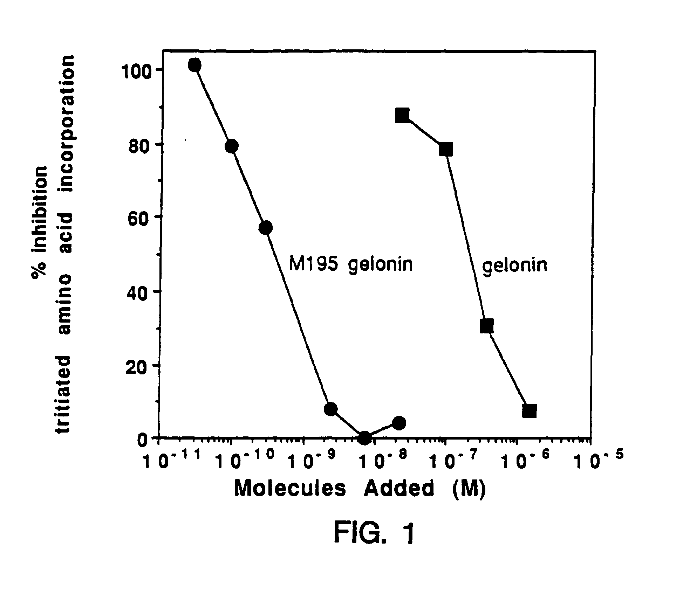

With reference to Figure 1, M195 immunotoxin was tested for its ability to kill HL60 cells in comparison to free gelonin. Inhibition of protein synthesis (using the test of a tritiated mixed amino acid (0.5 µCi/ml; New England Nuclear Corp.) incorporation into trichloroacetic acid (TCA) precipitable protein) was used as a measure of activity of the agent used. Final concentrations of the ranged M195-gelonin immunotoxin from 4 µg per ml to 5 nanograms. Gelonin final concentrations ranged from 44 µg per ml to 0.6 µg per ml.

The M195-gelonin immunotoxin was approximately 600 times more potent than the free gelonin alone. ID₅₀ for the M195-gelonin immunotoxin was approximately 0.4 nM. Inhibition of protein synthesis by the M195-gelonin immunotoxin subsequently leads to either lack of cell division or to cell death. Cell death was confirmed by experiments using trypan blue exclusion to determine total number of live cells and percentages of live cells. With reference to Figure 1, HL60 cells with a final concentration of 1 x 10⁶ cells/cc were incubated for three days at 37°C in the presence of M195-gelonin immunotoxin or gelonin alone.

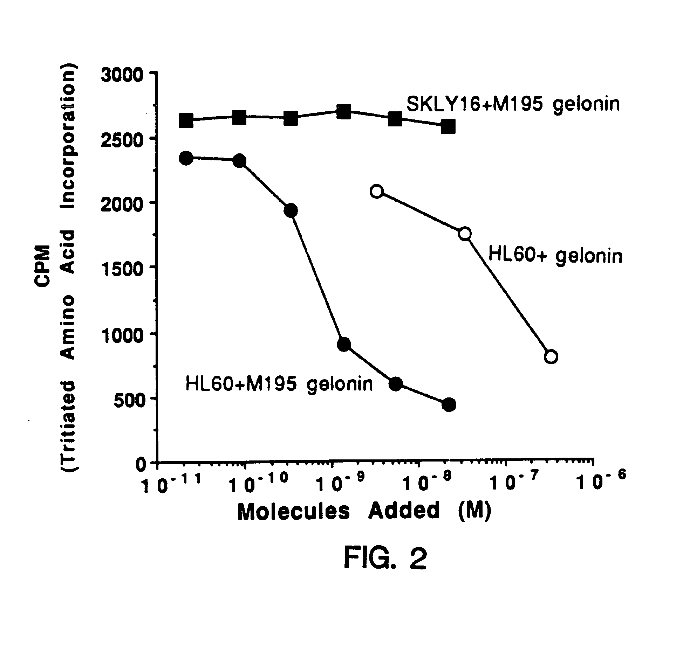

With reference to Figure 2, HL60 SKLY16 cells had a final concentration of 5 x 10⁵ cells/cc were incubated for three days at 37°C in the presence of either gelonin alone or the M195-gelonin immunotoxin. Final concentrations of M195-gelonin immunotoxin ranged from 4 µg per ml to 15.2 pg/ml. Gelonin final concentrations ranged from 10 µg/ml to 0.1 µg/ml. Levels of protein synthesis were determined by a five hour incorporation of tritiated amino acids into trichloroacetic acid precipitable protein.

As can be seen in Figure 2, concentrations of the M195 gelonin immunotoxin inhibited greater than 80% of HL60 protein synthesis; comparable concentrations of the immunotoxin had no effect on SKLY16 cells. Thus, the selectivity of the M195-gelonin immunotoxin is clear.

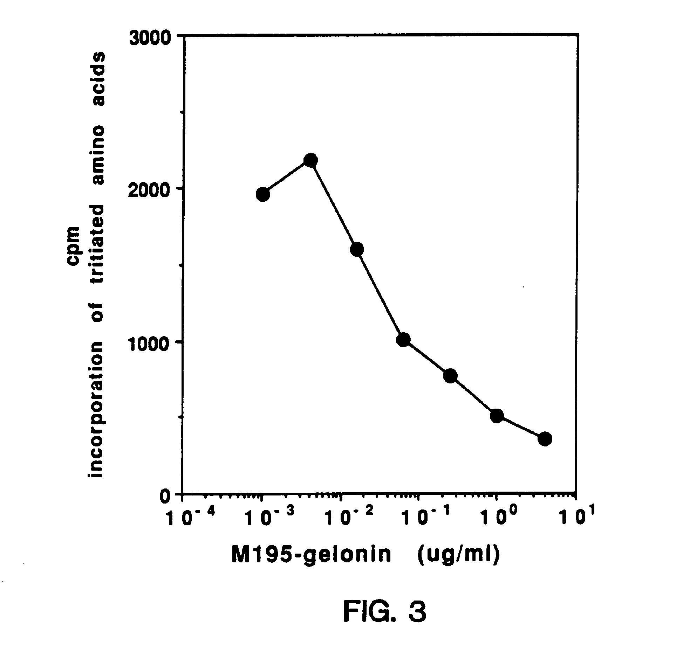

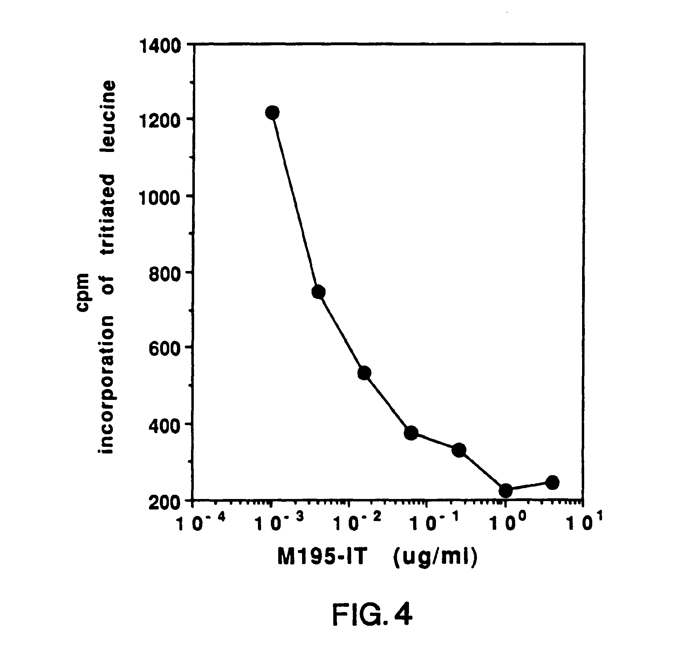

With reference to Figures 3 and 4, HL60 cells at a final concentration of 1 x 10⁶ were incubated for three days (Figure 3) or five days (Figure 4) at 37°C in the presence of the M195-gelonin immunotoxin. The final concentration of the immunotoxin ranged from 4 µg/ml to 0.9 ng/ml. Levels of protein synthesis were determined by a five hour incorporation of tritiated amino acids into trichloroacetic acid precipitable protein.

As is seen in comparison of Figures 3 and 4, the length of time of exposure to the immunotoxin affects its activity. In fact, an approximate ten-fold increase in potency of the M195-gelonin immunotoxin is seen after a five day incubation with HL60 cells as opposed to a three day incubation.

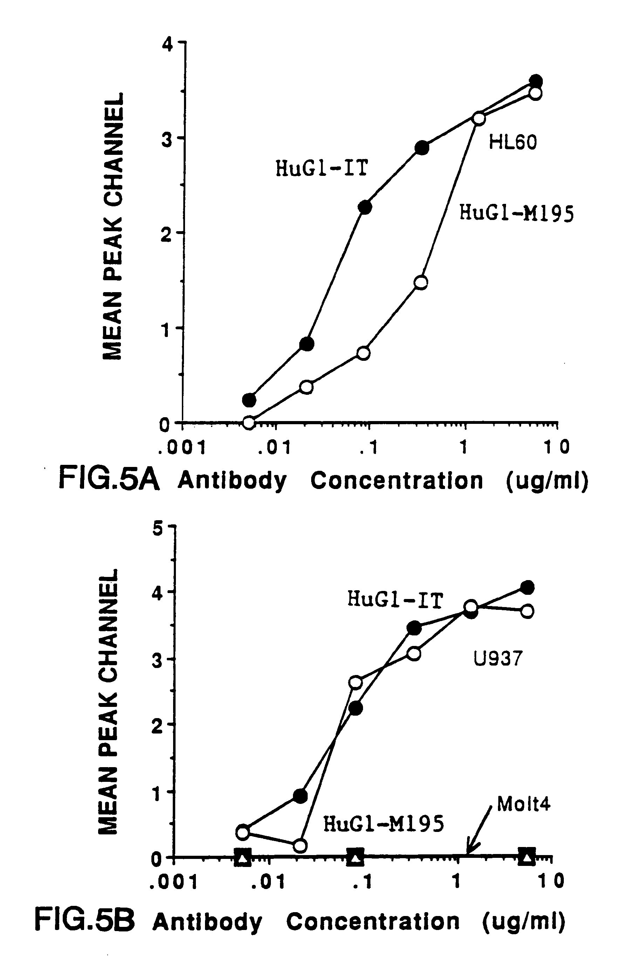

With reference to Figure 5, the binding of humanized M195 (HuG1)-immunotoxin to HL60, U937, and MOLT4 cells were examined. HuG1 and HuG1-immunotoxin were added to HL60 cells (Figure 5, panel A) or U937 and MOLT4 cell lines (Figure 5, panel B) in concentrations ranging from 5 µg/ml to 5 ng/ml. The cells were incubated in ice for one hour before excess antibody was washed away. Subsequently, goat antihuman FITC was added to cells and the cells were again incubated for one hour on ice. After washing away excess antibody, the cells were fixed at 0.5% paraformaldehyde and read on an EPICS Profile flow cytometer.

Figure 5 illustrates that the humanized M195-gelonin immunotoxin is capable of binding specifically to target cells. Using indirect flow cytometry, the humanized M195-gelonin immunotoxin showed more specific binding in Figure 5 to CD33 positive cell lines (HL60) and U937 comparable to the humanized M195 antibody alone. It did not bind to CD33 negative cell lines (MOLT4; Figure 5, panel B).

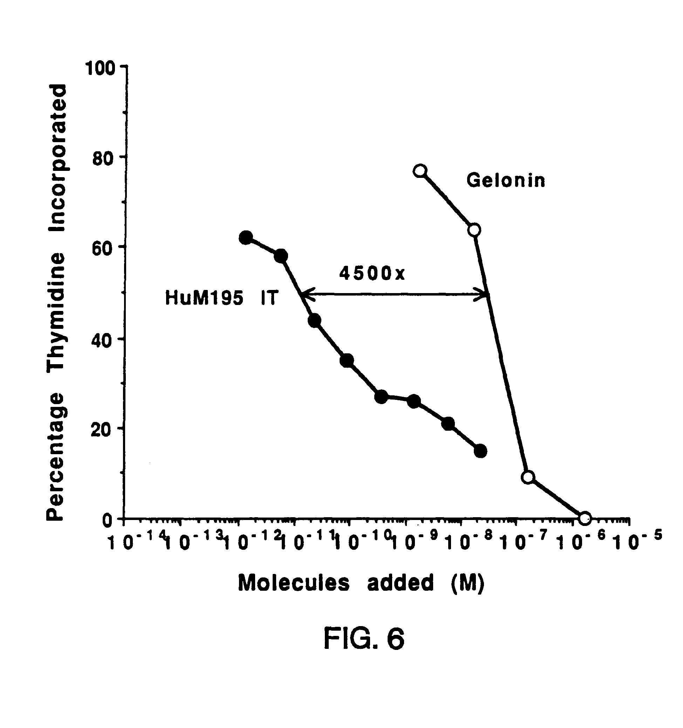

With reference to Figure 6, HL60 cells at a final concentration of 3 x 10⁴ and 5 x 10⁴ were incubated for five days at 37°C in the presence of the humanized M195-gelonin immunotoxin at a final concentration range of 4 µg/ml to 0.2 ng/ml. Gelonin final concentrations ranged from 50 µg/ml to 0.5 µg/ml. DNA synthesis was determined by a five hour incubation with tritiated thymidine. As illustrated in Figure 6, the humanized M195-gelonin immunotoxin inhibited protein synthesis approximately 4500 x that seen with gelonin alone.

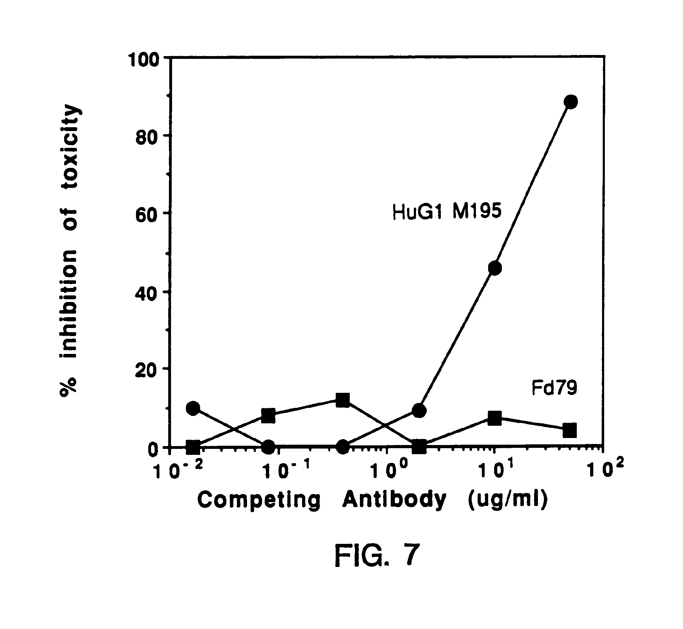

Figure 7 illustrates the ability of humanized M195-gelonin immunotoxin to kill HL60 cells. As is seen in Figure 7, the ID₅₀ for the humanized M195-gelonin immunotoxin was less than 10 picomoles. This ID₅₀ for the immunotoxin is more than 4000 times lower than the ID₅₀ of gelonin alone.

With reference to Figure 8, HL60 cells were incubated for one hour on ice in the presence of the humanized M195 antibody or F79, an isotype matched HuG1 control antibody. Humanized M195-gelonin immunotoxin was added at a concentration of 0.15 µg/ml. Cells were incubated 90 hours at 37°C. The quantity of live cells was determined by the trypan blue exclusion technique. The percent inhibition represents the reduction of cells killed in the presence of the competing antibody as compared to cells killed by the immunotoxin alone.

As seen in Figure 8, the humanized M195 antibody was able to block cytotoxicity of the humanized M195 antibody-gelonin immunotoxin in a dose dependent manner. In contrast, Fd79, a non-specific humanized IgG1, had no effect at the same levels.

In conclusion, therefore, it is seen that the present invention and the embodiments disclosed herein are well adapted to carry out the objectives and obtain the ends set forth at the outset. Certain changes can be made in the method and apparatus without parting from the spirit and scope of this invention. It is realized that changes are possible and it is further intended that each element or step recited in any of the following claims is to be understood as referring to all equivalent elements or steps for accomplishing substantially the same results in substantially the same or equivalent manner. It is intended to cover the invention broadly in whatever form its principles may be utilized. The present invention is therefore well adapted to carry out the objects and attain the ends and advantages mentioned, as well as others inherent therein.

## Claims

1. A composition comprising a protein exhibiting binding specificity for an antigen domain for CD33 protein and a plant derived toxin **characterised in that** the protein exhibiting binding specificity and the plant derived toxin are conjugated to one another and **in that** the plant derived toxin is selected from the group consisting of gelonin, full length recombinant gelonin and gelonin fragments.

2. A composition according to claim 1, **characterised in that** the binding specificity is for an extracellular epitope of CD33.

3. A composition according to claim 1 or claim 2, **characterised in that** the domain region is from a single chain antibody.

4. A composition according to any one of claims 1 to 3, **characterised in that** the domain region is selected form the group consisting of murine monoclonal antibodies and humanised monoclonal antibodies.

5. A composition according to any preceding claim, **characterised in that** the toxin is a native toxin or a recombinant toxin.

6. A composition according to any preceding claim, **characterised in that** the composition further comprises a pharmaceutically acceptable carrier.

7. The use of a composition according to any one of claims 1 to 6 in the preparation of a medicament for the treatment of neoplastic cell.

8. Use according to claim 7, **characterised in that** the cells are acute myeloid leukaemia cells, chronic myeloid leukaemia cells, acute myelodysplastic syndrome cells, chronic myelodysplastic syndrome cells, cells resulting from refractory anaemias, lymphoid leukaemia cells or undifferentiated leukaemia cells.

9. Use according to claim 7 or claim 8, **characterised in that** the medicament is for retardation of the growth of the cells.

10. Use according to any one of claims 7 to 9, **characterised in that** the medicament is to be used for the treatment of human or non-human animals.

11. Use according to any one of claims 7 to 10, **characterised in that** the neoplastic cell derives from a recurring neoplastic condition and **in that** the medicament prevents recurrence of the neoplastic condition.

12. Use according to any one of claims 7 to 11, **characterised in that** the medicament extends the survival time of the host animal.

13. Use of a composition according to any one of claims 1 to 7 for the treatment of *in vitro* neoplastic cells.

14. Use according to claim 13, **characterised in that** the neoplastic cell is in a mixed cell population.

15. Use according to claim 14, **characterised in that** the mixed cell population comprises bone marrow.

16. A composition comprising a protein exhibiting binding specificity for an antigen domain for CD33 protein and a plant derived toxin, **characterised in that** the protein exhibiting binding specificity and the plant toxin are a fusion protein and **in that** the plant derived toxin is selected from the group consisting of gelonin, full length recombinant gelonin and gelonin.
